# EUROPEAN PATENT SPECIFICATION

(11) **EP 4 331 471 B1**
(45) Date of publication and mention of the grant of the patent: **10.06.2026**
(21) Application number: 22193428.4
(22) Date of filing: 01.09.2022
(51) Int. Cl.: A61B 5/00, A45D 44/00

(54) **METHOD FOR DETERMINING A CONDITION OF THE INTERNAL STRUCTURE OF HAIR AND ASSOCIATED DEVICE**
VERFAHREN ZUR BESTIMMUNG EINES ZUSTANDES DER INNEREN STRUKTUR VON HAAREN UND ZUGEHÖRIGE VORRICHTUNG
PROCÉDÉ POUR DÉTERMINER UN ÉTAT DE LA STRUCTURE INTERNE DE CHEVEUX ET DISPOSITIF ASSOCIÉ

(43) Date of publication of application: 06.03.2024
(73) Proprietor: Henkel AG & Co. KGaA, 40589 Düsseldorf (DE)
(72) Inventor: Khadem, Behnam, 40764 Langenfeld (DE); Welzel, Jessica, 22547 Hamburg (DE); Grosjacques, Camille, 20255 Hamburg (DE); Malhotra, Aastik, 40589 Düsseldorf (DE); Koenen, Annika, 41515 Grevenbroich (DE); Bayersdoerfer, Rolf, 22589 Hamburg (DE)

(56) References cited:
- US-A1- 2019 192 067
- US-A1- 2019 216 387
- US-A1- 2021 204 865

## Description

### TECHNICAL FIELD

The invention pertains to the field of hair care and in particular to the field of measuring and determining the status of hair, in order to be able to decide on future hair care treatment and apply adapted hair care treatments that improve or maintain a certain hair condition that is also referred to as hair "health". The invention provides a method that is more particularly sensitive to an internal condition of hair and is capable of detecting the effect of previous treatments applied to hair, thereby enabling a personalized counseling for hair care treatments.

### TECHNOLOGICAL BACKGROUND

Hair is a biological multicomponent fibre with a high protein content of up to 95 weight percent. Structurally, the hair cross-section can be divided into two to three distinct morphological regions, those being from outer to inner regions: the cuticle, the cortex and medulla (the medulla being present in some hairs but not all). The protein part of the cortex is made up of α-keratin helixes embedded in a proteinaceous matrix.

Cosmetic products, such as for example shampoos, conditioners, treatment masks, colorations are used to improve or change hair properties in order typically to facilitate combing, improve the smoothness of the texture of hair, increase hair gloss as well as to reduce hair breakage or to give hair a new color.

The structure of hair differs from person to person due to the many factors that can affect the inner (cortex and medulla) and outer (cuticle) structure of hair. Most often, the hair of two different people will react differently to the same hair treatment. Hair care professionals require years of experience in order to assess the potential of different hair care treatments at improving a person's hair condition and/or at achieving a desired hair property (hair color, hair gloss, hair volume, hair smoothness, hair damage which can range from very damaged to not treated, not damaged; damage being sometimes referred to as an indicator of hair "health").

Even hair care professionals in salons can benefit from tools that assist them in obtaining information on the condition of hair of their clients in order to choose the most suitable hair treatments. In order to decide on products that can help improve hair condition or maintain at a minimum the damage caused to hair, the SalonLab^{®} Analyzer developed by Henkel^{®} provides valuable information that enables users (professionals as well as non-professionals) to determine the degree of damage of their hair by measuring a cysteic acid content of their hair with infrared and/or near infrared sensing. The infrared and/or near-infrared spectrum is compared to a calibration model in order to assess precisely the degree of damage of hair indicative of the hair "health" of the user. Further information on this approach can be found in WO2018/007353.

Additionally, US 2021204865 A1 describes an arrangement for determining a degree of stretching of hair. The arrangement includes a detection unit for detecting hair characteristics and an evaluation unit for evaluating the detected hair characteristics and for determining the degree of elongation of hair based on the detected hair characteristics.

Despite the huge potential of such a non-intrusive optical sensing approach, the calibration model is less efficient at providing useful information about the condition of the internal structure of hair in particular the state of the medulla and cortex. As a result, information is missing and it is challenging to provide a complete picture of the condition of hair.

This is noticeable when trying to objectively assess the effect of a hair treatment on the full structure of the hair, including the effect on the internal structure of the hair. Some hair treatments substitute broken bonds inside hair as well as on the outside (cuticle) of hair, although only the effect on the cuticle can be accurately investigated. It is difficult to estimate the exact efficiency of this improvement process without information regarding the state of the cortex and medulla and based only on the effect of the treatment on the cuticle.

A further challenge in understanding the full effect of hair treatments on hair is the influence of hair washing on the cuticle which tends to remove traces of the previous treatments applied to hair. Water tends to adsorb on the cuticle and removes traces of previous hair treatments. This affects the ability of existing methods to accurately detect the effect of previous hair treatments as information regarding the full picture on the effect of hair treatments on the cortex and medulla of hair fibers is missing.

For the above reasons, a method for determining a condition of the internal structure of hair is sought.

### SUMMARY OF THE INVENTION

To address the above need, the invention provides a method for determining a condition of the internal structure of hair, the method comprising:
- while exposing a hair sample of the hair to at least one among light, stress, elongation or heat, recording a signal from at least one part of the light that has interacted with the hair sample or a signal from the response of the hair sample to that stress, elongation or heat;
- comparing the recorded signal with a first calibration model and determining the condition of the internal structure of hair based on the comparison;
wherein the first calibration model is obtained by:
- determining the condition of the internal structure of a plurality of calibration hair samples using a method that measures *internal constituents or structure of hair fibers;*
- exposing each of said plurality of calibration hair samples to at least one among light, stress, elongation or heat, recording a signal from at least one part of the light that has interacted with each calibration hair sample or a signal from the response of the hair sample to that stress, elongation or heat;
- establishing the first calibration model by determining a correlation between the recorded signals and the determined condition of the internal structure of the plurality of calibration hair samples.

The method of the invention enhances previous methods for determining the status of hair of a user by giving access not only to the condition of the outer portion of hair (the cuticle) but instead and/or further by providing more accurate insights into the internal structure of hair (information regarding the state of the cortex and medulla, typically on top of the information also accessible on the state of the cuticle). The above method is therefore capable of providing a more complete picture of the condition of the structure of hair fibers of a user.

The calibration method that is used is typically one that measures bulk properties of the constituents of hair. The term "bulk properties of the constituents of hair" is to be understood as referring to the internal constituents or structure of hair fibers and not only molecular vibration modes that mostly are perceived on the outer layers of hair fibers (the cuticle). Such methods rely on analytical or biophysical methods that differ from the well-known chromatography and/or High Performance Liquid Chromatography (HPLC) method of the prior art. Bulk properties give insights on components also located below the cuticle. A non-limitative list of techniques that are sensitive to the inside of the hair fibers include stress-strain measurements, fatigue measurements, elongation measurements, hair hydrophobicity or swelling behaviour, measurements of non-linear optical effects arising in hair such as birefringence of waves, attenuated total reflectance, Fourier Transform Interferometry, differential scanning calorimetry to name a few.

The calibration model is established by measuring with precision the condition of the internal structure of hair using one of such techniques on "calibration" hair samples. The same calibration hair samples are then investigated using a less sophisticated measurement technique, that can either still include one of the above techniques, or as is also particularly advantageous, a non-intrusive optical measurement using either infrared (IR), near-infrared (NIR), hyperfrequency spectrum analysis or image analysis.

The method of the invention arises from the observation that such simple and non-intrusive methods comprise traces of information regarding the condition of the internal structure of hair fibers. Such traces cannot be seen in the form of a specific absorption at certain wavelengths of the spectrum that would be documented in literature. Without the intention of being bound to theory, Applicant assumes that these signals comprise indirect effects of the internal structure of the hair that may also reflect on the cuticle in unexpected ways. Therefore, the existing IR and NIR sensing technique used in the SalonLab^{®} Analyzer developed by Henkel^{®} are also capable of determining the condition of the internal structure of hair using a different calibration model established on the basis of techniques that measure bulk properties of the constituents of hair.

The term "condition of the internal structure of hair" is to be understood as meaning "condition of at least the internal structure of hair" as the method of the invention can be sensitive to both the internal structure of hair and the outer structure of hair, thereby providing a more complete information regarding the overall condition of the structure of the hair of a user.

According to an embodiment, the condition of the internal structure of hair is at least one among: the degree of hair damage, the presence of external chemical compounds that are on or in hair, the presence and type of chemical compounds that are on or in hair, a moisture content of hair.

Applicant observes that the calibration model established according to the method described above provides insights into the condition of the internal structure of hair fibers and in particular a more complete state of the extent and degree of hair damage than prior art methods. It can also detect the effect of or the presence of chemical compounds that are in or on the hair fibers from previous hair treatments or from exposure to the environment. The calibration method is also efficient at determining the moisture content of hair fibers with more accuracy than prior art methods.

According to an embodiment, the independent method that measures internal constituents of hair fibers is at least one among: differential scanning calorimetry applied to hair, a mass spectrometry of hair, electron microscopy of hair, stress-strain measurements, fatigue measurements, elongation measurements, hair hydrophobicity or swelling detection, measurements of non-linear optical effects arising in hair such as birefringence of waves, attenuated total reflectance, Fourier Transform Interferometry.

The above list of techniques give access to the internal structure of hair fibers and thereby further contribute to the establishment of a more sophisticated calibration model than prior art methods.

According to an embodiment, the recorded signal is at least one among: an infrared light spectrum, a near-infrared light spectrum, a combination of wavelengths forming a hyperspectral signal, a Fourier Transform Interferometry signal, an image of hair, a differential scanning calorimetry applied to hair, stress-strain measurements on hair, elongation measurements conducted on hair, a birefringence signal scattered by hair, a signal related to a vibration of hair, a signal related to the response of hair to ultrasounds, a pH value measured on hair, an electrical conductivity of hair, a signal related to surface charge of hair.

As mentioned above, the signals recorded on the hair of users may be of the same nature as the signals recorded to establish the calibration model. However, one particularly advantageous aspect of the invention lies in the fact that a simple and non-intrusive non-destructive investigative method such as image analysis, hyperfrequency spectrum analysis or the IR or NIR detection method of the SalonLab^{®} Analyzer can be used in the method of the invention and give insights into the condition of the internal structure of hair fibers with the improved calibration model.

According to the invention, the method further comprises:
- obtaining a second calibration model that establishes a correlation between a determined degree of hair damage of the calibration hair samples and signals recorded from at least one part of the light that has interacted with calibration hair samples or signals corresponding to a response of hair to stress, elongation or heat while exposing calibration hair samples of the hair to that light, stress, elongation or heat.

The second calibration model typically relies on information that is already used in prior art in the Salonlab^{®} Analyzer device developed by Henkel^{®}. It is a calibration model conceived to detect the degree of damage of hair in particular through a determination of a content of cysteic acid in hair, and more particular in the cuticle of hair. According to an embodiment, the degree of hair damage is determined using at least one among High Performance Liquid Chromatography and a chromatographic method that determines a degree of cysteic acid content of the calibration hair samples.

According to the invention, the signal recorded while exposing the hair sample of the hair to at least one among light, stress, elongation or heat is compared to the second calibration model to determine a degree of hair damage of the hair sample.

Such a comparison provides the possibility to distinguish between the contribution of the cuticle to the condition of hair fibers and the contribution of the cortex and medulla to the condition of hair fibers. A subtraction of the signal acquiring using both calibration models provides insights into this specific contribution of the internal part of the structure of the hair fiber to the condition of hair. This internal part typically comprises stronger markers of the previous hair treatments applied to hair which provides a means to determine how effective a hair treatment is at improving an overall condition of hair of a user. This constitutes a valuable tool for salon professionals and individuals alike in understanding the effect of hair treatments on the hair of users and adapt these treatments to the quantitatively assessed condition of the hair.

In particular, the above method further comprises:
- determining a first value of the condition of the internal structure of hair and a first value of the degree of hair damage prior to applying a hair treatment to the hair;
- determining a second value of the condition of the internal structure of hair and a second value of the degree of hair damage after application of the hair treatment;
- comparing the difference between the second value of the degree of hair damage and the first value of the degree of hair damage to the difference between the second value of the condition of the internal structure of hair and the first value of the condition of the internal structure of hair;
- determining, based on said comparison, a specific contribution of the application of the hair treatment to the condition of the hair.

Such an approach is one way to determine the specific contribution of the application of a hair treatment to the condition of hair of a user.

The term "hair treatment" typically refers to at least one among: application of a hair product such as a hair dye, a hair bleaching agent, a shampoo, a conditioner, a hair volume enhancing agent, a hair gloss enhancing agent, a hair perming treatment, a hair ironing treatment, cutting the hair, adding hair extensions to the hair 5 of the user. Preferably a "hair treatment" refers to the application of a product such as a shampoo, a conditioner or a hair bleaching agent.

According to another embodiment, the method further comprises:
- determining a relationship between values of the condition of the internal structure of hair obtained using the first calibration model and values of the degree of hair damage obtained using the second calibration model;
- determining a value of the condition of the internal structure of hair and a value of the degree of hair damage after application of the hair treatment;
- using the determined relationship to transform the value of the degree of hair damage into a corresponding equivalent value of the condition of the internal structure of hair;
- subtracting from the determined value of the condition of the internal structure of hair the corresponding equivalent value to estimate the additional contribution of the application of the hair treatment on the value of the condition of the internal structure of hair.

According to another embodiment, the method further comprises:
- determining a first value of the condition of the internal structure of hair prior to applying a hair treatment to the hair;
- determining a second value of the condition of the internal structure of hair after application of the hair treatment;
- comparing the difference between the second value of the condition of the internal structure of hair and the first value of the condition of the internal structure of hair;
- determining, based on said comparison, a specific contribution of the application of the hair treatment to the condition of the hair. Such approaches are alternatives to the determination of the specific contribution of the application of a hair treatment to the condition of hair of a user.

According to an embodiment, the method further comprises:
- providing a recommendation of a hair treatment based on the determined condition of the internal structure of hair, the recommended hair treatment being selected from a database associating hair treatments to a quantified effect of the hair treatment on improving a condition of hair after application of said hair treatment based on an initial condition of the internal structure of hair.

The invention also pertains to a device for determining a condition of the internal structure of hair, according to claim 10.

Such a device can typically be used an enhanced version of the Analyzer device developed by Henkel^{®} for SalonLab^{®}. Such a device may typically further comprise a data storage device which stores information relating to the first calibration model and optionally also the second calibration model. This information can also be received via a communication device which receives information from a distance storage unit such as a server computer or cloud.

According to an embodiment, the detector comprises at least one among: an infrared sensor, a near-infrared sensor, a sensor capable of acquiring or reconstructing a hyperspectral signal, a heat capacitance sensor configured to acquire a differential scanning calorimetry signal, a camera configured to acquire an image of the hair. According to an embodiment, the device is at least one among: a handheld device configured to grasp a strand of hair between two portions of the handheld device, a mobile terminal.

The method of the invention can according to some embodiments be implemented not only using the handheld analyzer device, but directly by running a program such as an "App" on a smartphone or any other portable device or a computer equipped with a detector as described above. This is in particular possible since instead of IR or NIR signals, the method of the invention can rely on hyperspectral signals that can also be acquired using mobile phones, in particular when reconstructing a hyperspectral spectrum using available LED combinations from mobile phones and using a suitable algorithm as is already known from prior art methods, for example that explained in WO2018/228859.

The invention also pertains to a computer program product according to claim 13.

### BRIEF DESRIPTION OF THE DRAWINGS

The present disclosure will hereinafter be described in conjunction with the following drawing figures, wherein like numerals denote like elements, and:
Fig. 1 is a schematic illustration of the implementation of a method according to an embodiment on the hair of a user using a device according to an embodiment;
Fig. 2 is a flowchart illustrating a method according to an embodiment;
Fig. 3 is a flowchart illustrating a further aspect of an embodiment;
Fig. 4 is a schematic illustration of a device according to an embodiment;
Fig. 5a, 5b and 5c are schematic illustrations of a user interface implementing aspects of an embodiment.

### DETAILED DESCRIPTION

The invention pertains to a method for determining the internal structure of the hair of a user in order to acquire a more complete understanding of the true state of a user's hair. This in turn enables a better decision on the type of hair treatment that can either maintain the condition of the hair or improve that condition. The method of the invention can be used by professionals in salons to assisting them in understanding in a more objective and quantitative manner the state of the hair of their clients. The method of the invention can also be implemented by individual users. The method can be implemented on a portable device or run on a mobile device and be used together with a computer program (for example an app).

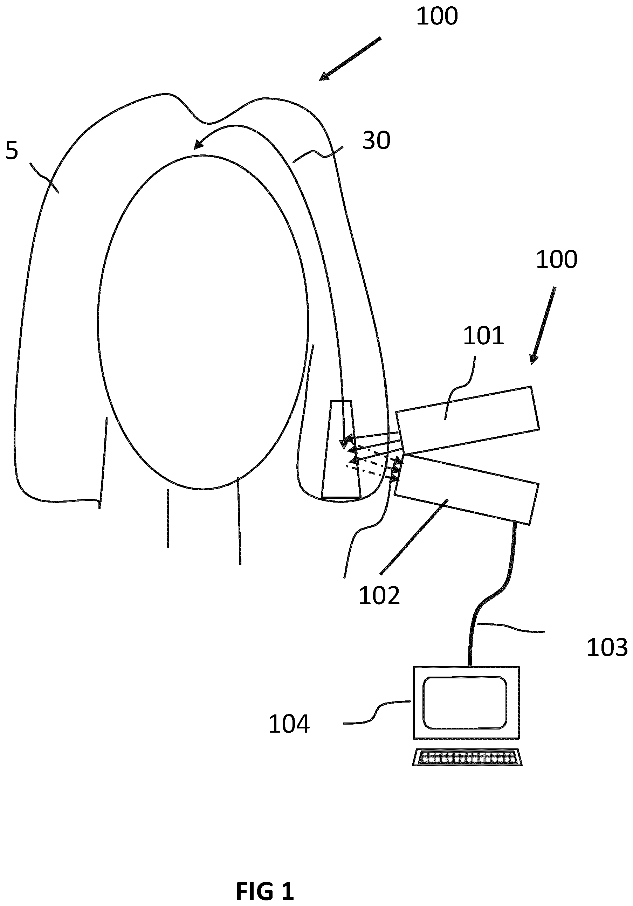

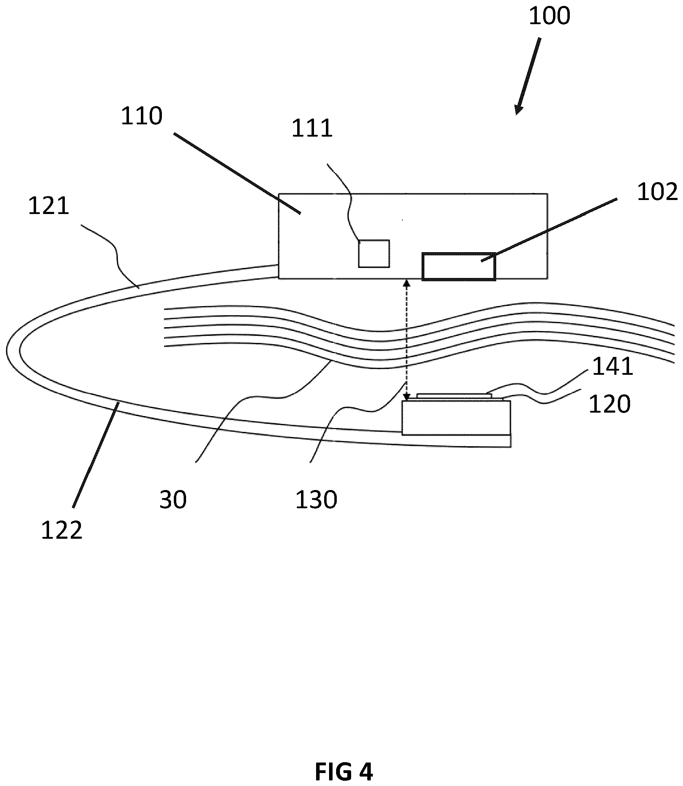

Figure 1 provides a schematic representation of a device 100 usable in implementing a method according to the invention. A further embodiment of that device 100 is illustrated on figure 4 which will be commented hereunder. Hair of a user and more particularly a hair sample 30 is subjected to a stimulus and the response of hair to that stimulus is measured with a detector 102. In the example represented on figure 1 the device 100 is a handheld device that uses an optical signal to determine the condition of the internal structure of hair 5 of the user. The device 100 comprises a light emitter 101 that emits light for example in the infrared and/or near infrared spectral ranges. The infrared spectral range typically comprises wavelengths between 700 nm and 1 mm. The near infrared spectral range typically comprises wavelengths between 700 nm and 2500 nm. Infrared and near infrared are particularly advantageous spectral ranges for observing physical objects because they can be analyzed in a non-intrusive, non-destructive way and provide insights into vibrational modes of molecules forming the object that is investigated.

Vibrational modes of molecules can in particular be used to measure the degree of hair damage via the amount of cysteic acid that is present in hair. Indeed, cysteic acid is a marker of broken dissulfide bonds (S-S) in hair and is an accurate indicator of the damage state of hair.

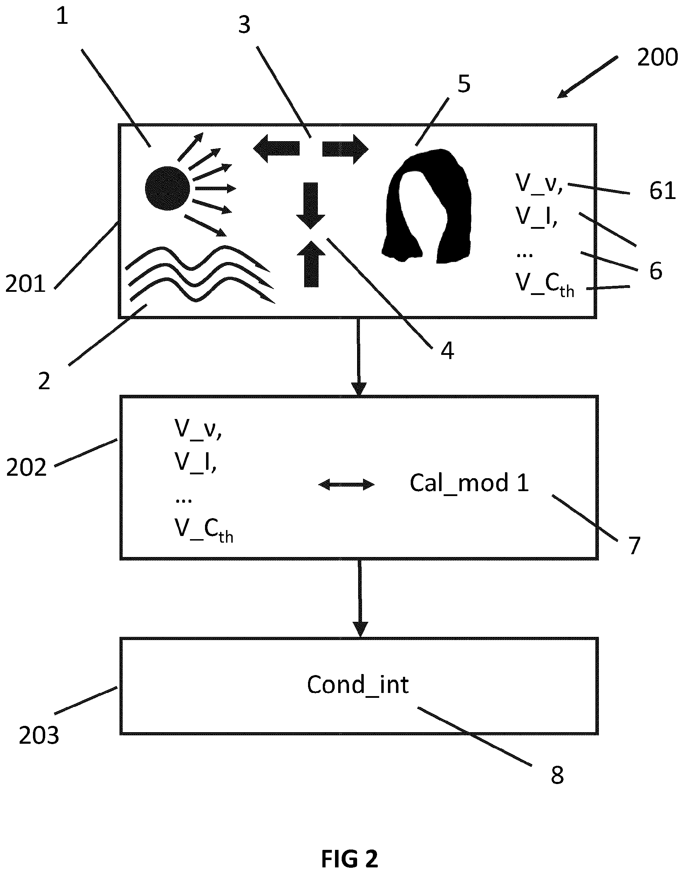

Apart from infrared or near-infrared sensing, the device 100 may rely on other optical sensing technologies such a FTIR (Fourier Transform Interferometry), ATR (attenuated total reflectance), hyperspectral imaging (for example either by using a hyperspectral camera or by reconstructing a hyperspectral signal from several narrow band signals recorded from hair acquired under different illuminates for example those that can be generated with a portable phone as described for example in WO2018/228859. It is also possible to acquire information regarding the condition of the hair 5 of the user using an imaging device in the visible spectral range. Such image analysis tools can detect defects on hairs provided the image has a high enough resolution. Detection of the defects in the image can be determined based on a detection algorithm trained using a plurality of images of hair of known damage state using a calibration model such as one that is presented in connection with figure 2 hereunder.

The device 100 may also be replaced by a mobile phone and have different shapes that are not represented on the figures. Some shapes of the device 100 can for example be more suitable to access the root, length and tips of hair and differ from the V or U-shaped device represented on figures 1 and 4.

Figure 1 further illustrates that the device 100 may communicate either wirelessly or in a wired 103 manner with a remote data processing device such as a computer 104, a cloud service or server. The device 100 may also comprise computing means in the device itself.

The invention also pertains more particularly to a method 200 such as schematically illustrated on the flowchart of figure 2. This method 200 typically comprises a step of recording 201 a signal 61 from at least one part of the light that has interacted with the hair sample 30 while exposing a hair sample 30 of the hair 5 to light 1. As seen on figure 2, other means of recording a signal can be implemented such as for example exposing the hair sample 30 of hair 5 to one among stress 4 (such as mechanical stress, shear stress, compressive stress, torsional stress), elongation 3 or heat 2. The response of the hair sample 30 of hair 5 to such an input is then recorded in the form of a signal 6. The heat response of hair 5 can be recorded in a differential scanning calorimetry measurement. In such an experimental setup, hair samples 30 are exposed to heat in order to determine the amount of heat required to increase the temperature of the hair sample.

This gives access to the heat capacitance of the hair sample 30 which can provide insights into the condition of the internal structure of the hair sample 30.

The most preferred embodiment consists in recording a signal 61 corresponding to light that has interacted with the hair sample 30, in particular infrared and/or near-infrared light.

Then, the recorded signal 6, 61 is compared 202 to a first calibration model 7. The calibration model 7 is for example a chart or mathematical function that provides a correspondence between a value of the recorded signal 6, 61 and a value of the condition of the internal structure 8 of the hair sample 30.

Based on this comparison, the condition of the internal structure 8 of the hair sample 30 is determined 203 by identifying the value within the calibration model 7 that typically represents a closest match to the value of the recorded signal 6, 61.

One strength of the method of the invention relies in the ability to determine this value of the internal structure 8 of the hair sample 30 even with sensing techniques that are not in principle capable to directly have access to such an internal structure (that is to say, the structure of the hair 5 below the cuticle). One aspect of the invention relies in the establishment of the first calibration model 7 used in the method 200 of the invention.

The first calibration model 7 is established by measuring the condition of the internal structure 8 of calibration hair samples. The value of the condition of the internal structure 8 of these calibration hair samples is known either because the hair was exposed to an event that caused the condition of the internal structure 8 to reach a predetermined value, or this value is measured using a method that measures *internal constituents or structure of hair fibers.* Such a method is typically one which is sensitive to the internal structure of the hair 5 below the cuticle and can involve one among: differential scanning calorimetry applied to hair, a mass spectrometry of hair, electron microscopy of hair, stress-strain measurements, fatigue measurements, elongation measurements, hair hydrophobicity or swelling detection, measurements of non-linear optical effects arising in hair such as birefringence of waves, attenuated total reflectance, Fourier Transform Interferometry.

The same calibration hair samples are then investigated using a different method. Advantageously, this other method is non-intrusive, non-destructive and relies on optical sensing means. Preferentially the other method is an infrared and/or near-infrared sensing technique that measures an infrared and/or near-infrared spectrum of light that has interacted with hair while exposing hair to such light. Alternatively, the type of signals recorded that were mentioned above (exposing the hair sample 30 of hair 5 to one among stress 4 such as mechanical stress, shear stress, compressive stress, torsional stress, elongation 3 or heat 2 and recording the response of the hair to such stimulus) may also be used in this other method.

Since the calibration hair sample necessarily has the same value for the condition of the internal structure 8 thereof when measured using these two different methods, both values determined using the two different measurement techniques can be compared to establish a calibration curve.

According to a preferred embodiment, the values of the condition of the internal structure 8 of the calibration hair samples is determined using differential scanning calorimetry. Then the condition of the internal structure 8 of the calibration hair samples is measured using an infrared and/or near-infrared sensing technique such as one already known in connection with the SalonLab^{®} Analyzer developed by Henkel^{®}.

The correspondence established between the values acquired using the optical detection means and the differential scanning calorimetry measurements provides a way to determine the condition of the internal structure 8 of hair samples 30 using a simple non-intrusive and non-destructive method on any hair samples of any user. Another advantage of the use of an optical detection method is that it is faster to implement than other mechanical methods or methods relying on differential scanning calorimetry.

The method 200 of the invention enables a more complete and more objective and accurate quantitative determination of the state of the hair 5 of a user. This can be used in particular to determine the effect of hair treatments on the hair 5 of a user, and further used to recommend more customized and more suitable hair treatments that take into consideration the measured effect of the treatments on the hair 5 of users, each user having hair that may be in a specific condition due to the history of treatments applied to hair and biological or behavioral specificities of the user.

Several approaches may be implemented in order to extract from the method 200 presented above, the specific contribution of a hair treatment on the condition of hair 5 of a user.

One approach consists in taking a first measurement of the value of the condition of the internal structure 8 of the hair 5 of a user before a hair treatment is applied to said hair 5. Then, another measurement of the value of the condition of the internal structure 8 of the hair 5 of the user is made after application of the hair treatment to said hair 5. Since both measurement techniques are sensitive to the internal structure of the hair 5 of the user, the value of the first measurement can be subtracted from the value of the second measurement in order to extract the contribution to the value of the condition of the internal structure 8 of the hair 5 of the user attributable only to the hair treatment itself.

The term "hair treatment" typically refers to at least one among: application of a hair product such as a hair dye, a hair bleaching agent, a shampoo, a conditioner, a hair volume enhancing agent, a hair gloss enhancing agent, a hair perming treatment, a hair ironing treatment, cutting the hair, adding hair extensions to the hair 5 of the user. Most preferably "hair treatment" refers to the application of hair care products selected from a hair dye, a hair bleaching agent, a shampoo, a conditioner, a hair volume enhancing agent, a hair gloss enhancing agent.

Another approach consists in using two different calibration models in processing the same measurement of the value of the condition of the internal structure 8 of hair 5 of a user.

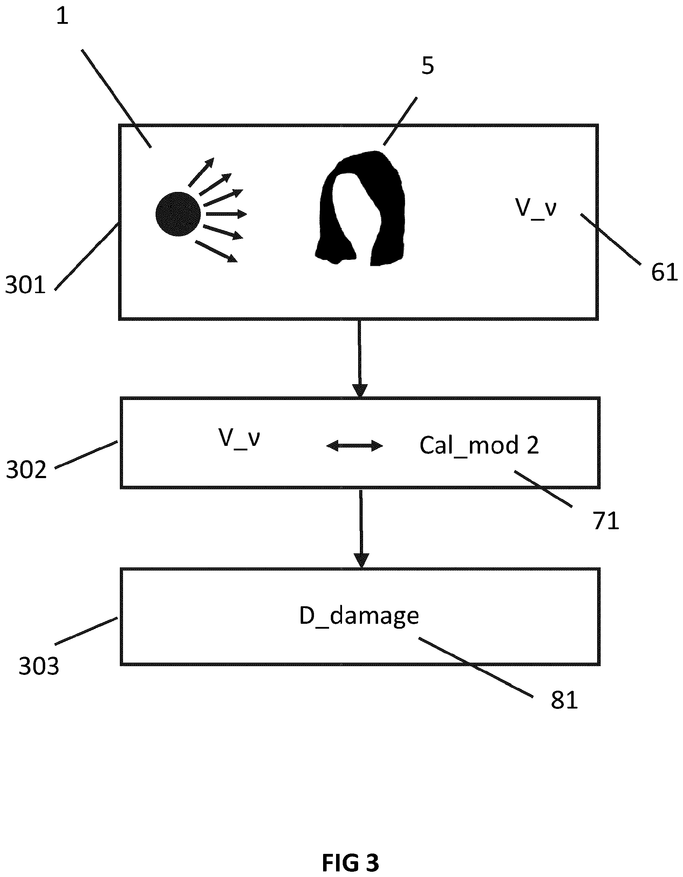

Figure 3 provides another schematic flowchart which represents steps of a method used in determining the degree of hair damage 81 using a second calibration model 71. The process represented on figure 3 corresponds to the state of the art already known and described for example in WO2018/007353. The process of figure 3 also comprises a step of recording 301 a signal corresponding to light 1 that has interacted with hair 5 of a user. The recorded signal 61 is compared 302 to a second calibration model 71. Based on this comparison, the degree of hair damage 81 is determined 303.

The second calibration model 71 is established using the approach described for example in WO2018/007353. Calibration hair samples are investigated using chromatography or HPLC (High Performance Liquid Chromatography) in order to determine their degree of hair damage which is also proportional to the cysteic acid content of the calibration hair samples. The same content of cysteic acid is determined using infrared and/or near-infrared sensing of the calibration hair samples. A correlation is established between the optical signal that is acquired using infrared and/or near-infrared sensing and the signal determined using HPLC or chromatography to build the second calibration model.

As opposed to the first calibration model 7, the second calibration model 71 is sensitive essentially to the degree of damage 81 of the cuticle of the hair 5. This provides a way to extract the contribution of a hair treatment to a value of the condition of the internal structure 8 of hair 5 from a single optical measurement of the signal 61 recorded from light 1 that has interacted with hair 5 or from any other measurement acquiring signals 6 as described above. It has been observed that there is a substantially linear dependency between the evolution of the degree of damage of hair 5 detectable using the second calibration model 71 and the evolution of the condition of the internal structure 8 of hair 5 detectable using the first calibration model 7. Establishing this correlation, it is possible to identify the specific contribution of the hair treatment to the value of the condition of the internal structure 8 of hair 5 by determining the value of the condition of the internal structure 8 of hair 5 using the first calibration model 7, determining the value of the degree of hair damage using the second calibration model 71 , using the correlation to transform the value of the degree of hair damage into a corresponding equivalent value of the condition of the internal structure of hair and subtracting the corresponding equivalent value from the determined value of the condition of the internal structure 8 of hair 5.

Figure 4 provides another schematic illustration of the device 100 that can implement the above method 200. The device might be a substantially U-shaped or V-shaped device 100 as illustrated on figure 4 but may also comprise other shapes, in particular linear shapes or substantially longitudinal devices in order to better have access to the roots, lengths and tips of hair 5.

The device 100 may typically comprise two portions 122, 121 between which a strand of hair can be placed such as hair sample 30. The spacing 130 between the two portions 122, 121 can be adjusted by pressing on the device in order to firmly and temporarily grasp the hair sample 30 for measurement purposes. The device 100 may have a base 120 that can comprise a dark covering 141 that helps placing the hair sample 30 below a detector 102 for measurement purposes. The dark covering 141 may also be used to calibrate the device 100 before starting a measurement and to remove the contribution from any local light interferences. The portion 121 that is opposite the base 120 may typically comprise some electronic components arranged in a distal end portion 110 of the device 100. Among other components, a data processing device 111 that is capable of acquiring the signal 6, 61 from the detector and process it in order to determine the value of the condition of the internal structure 8 of the hair sample 30 may be present in this distal end portion 110. The data processing device 111 may also comprise means for sending the measured signal 6, 61 to an external data processing structure such as a computer service or cloud architecture. Other arrangements of the different elements that constitute the device 100 may also be implemented.

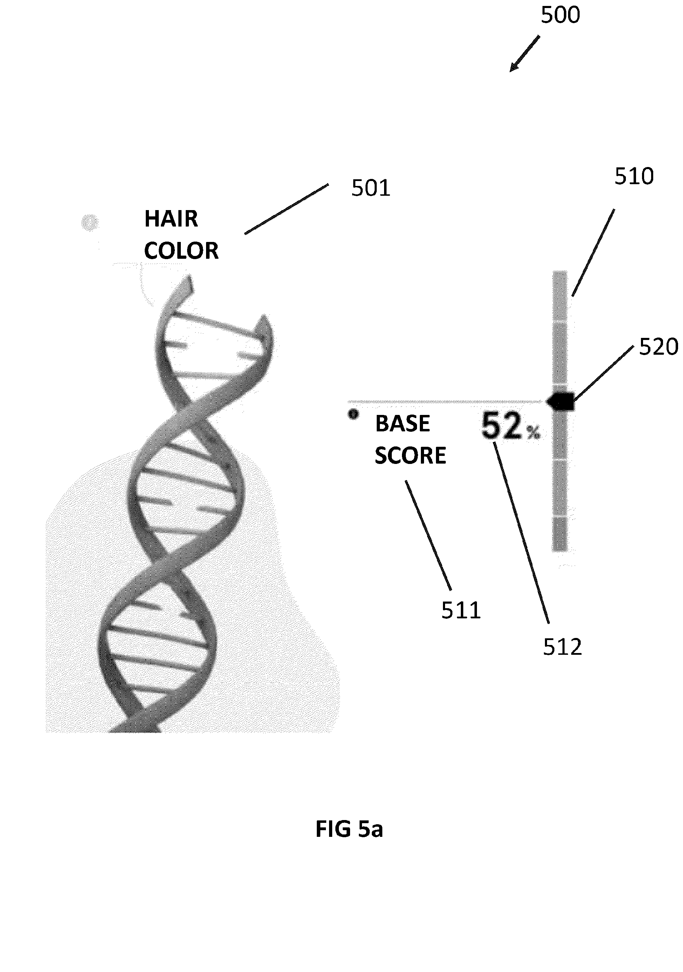

The method 200 can be implemented by a computer program that can be run on any terminal in the form of an app or any other user interface as schematically represented on figures 5a to 5c. Figures 5a to 5c further illustrate how the method 200 of the invention may display measurements in a format that is understandable to a user.

As seen on figure 5a, the user interface 500 can indicate information such as the hair color 501 of the user and provide a scale 510 on which values of the condition of hair are represented from very poor (at the lower end of the scale 510) to very good (at the upper end of the scale 510). On figure 5a, the scale 510 is subdivided into 5 different qualitative ranges or hair score categories. Each value of the condition of hair is associated with a score provided as a number between 0 (very poor condition) to 100 (very good condition). On Figure 5a, a base score 511 is provided. Base score 511 indicates that the measurement corresponds to an initial condition of the internal structure of hair 5 of the user, prior to application of any hair treatment.

Naturally, most users already exposed their hair to some hair treatment, be it by exposure to UV light by being exposed to sunlight, temperature changes, air pollution, previous application of hair care products. The base score 511 is an approximation of an "initial" (or untreated) hair condition, even though in reality the past history of the hair 5 of the user is already detectable in the base score 511 with the method 200 of the invention.

The numerical value 512 of that base score 511 is provided on the scale 510 and associated with a position 520 on the scale 510 that belongs to one of the five hair score categories. The scale 510 is an arbitrary unit scale that is representative of the degree of hair damage or hair score of the hair 5 of the user. This hair damage may reflect either only the damage as measured at the cuticle of hair 5 using the second calibration model 71 or the more complete hair damage as determined using the first calibration model 7.

User interface 500 may further comprise a questionnaire that collects further information from a user, in particular information regarding past hair treatments in order to adjust the value of the base score 511.

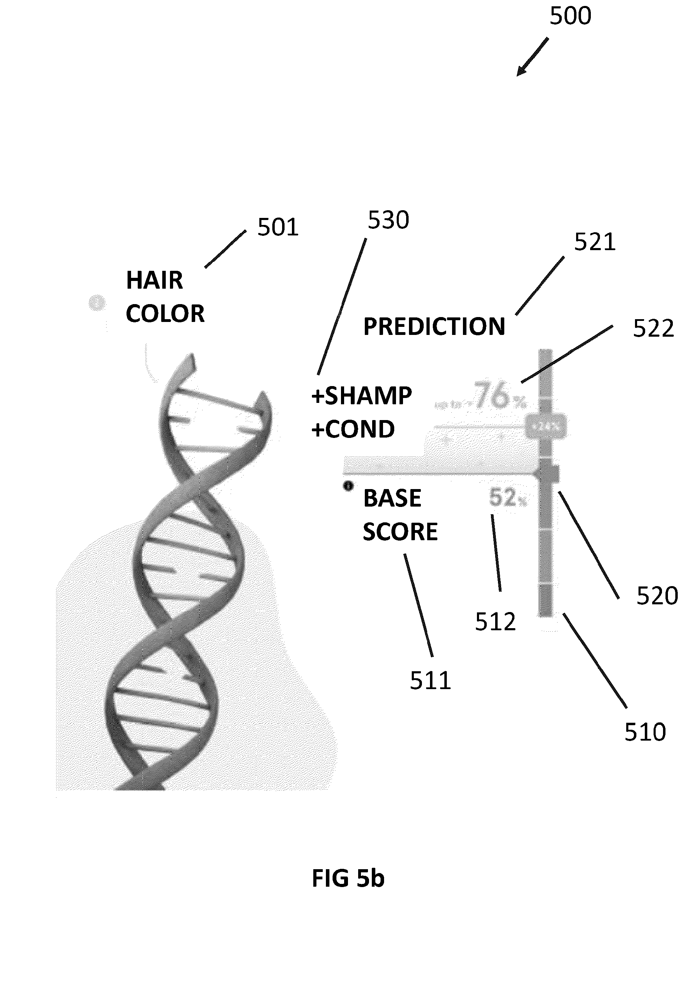

As seen on figure 5b, the user interface 500 may further comprise a recommendation of a hair treatment based on a prediction 521 of the achievable hair score 522 when applying a hair treatment 530 such as a shampoo and/or a conditioner to the hair 5 of the user. Such a prediction may typically be possible based on a database that associates hair treatments to a quantified effect of the hair treatment on improving a condition of hair after application of said hair treatment, wherein the hair to which it is applied has a certain initial condition of the internal structure of hair 5.

The prediction 521 may not fully reflect the true hair score that a user will achieve when applying the hair treatment 530, since the base score 511 may already include the effect of previous known or even unknown hair treatment to which hair 5 of the user was exposed. Furthermore, hair 5 of users being dependent on many different factors, it is likely that two different users having a similar base score 511 will in fact react differently when the same hair treatment 530 is applied to their hair.

The method 200 of the invention can assist in determining such differences and apply corrective measures to iteratively make better and better recommendations for future hair treatments 530 based on a measured evolution of the hair score.

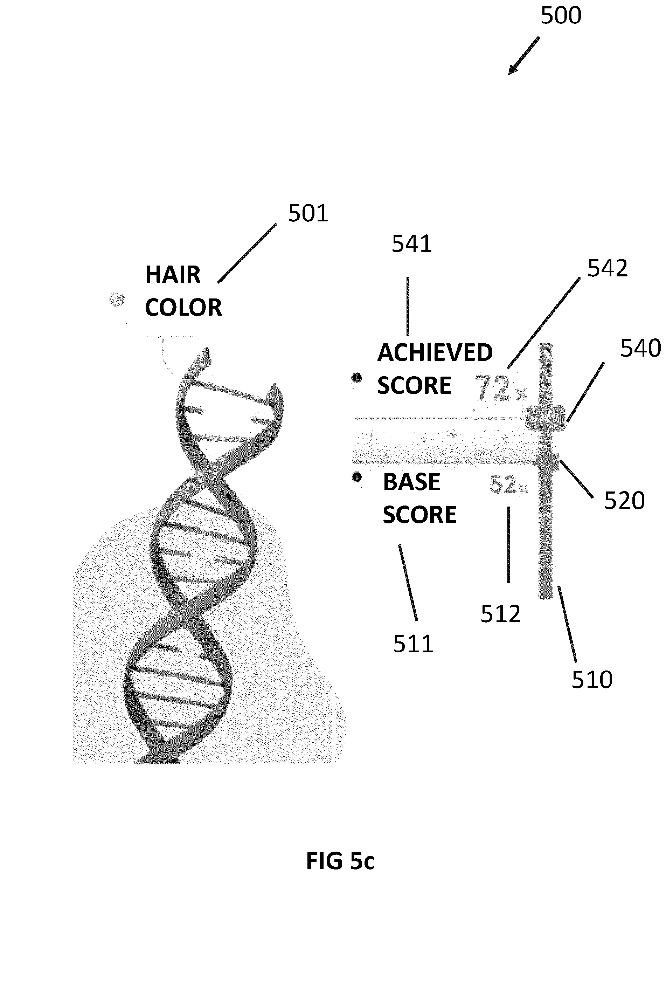

Figure 5c schematically illustrates the achieved score 541 measured on the hair 5 of a user after application of the hair treatment 530 recommended in figure 5b. One observes that the achieved hair score 542 has a value that is slightly lower than the one predicted in figure 5b. This difference can be used to determine facts regarding the effect of past treatments on the base score 511. Figure 5c is further evidence that the method of the invention 200 is capable of quantifying the effect 540 of the application of a hair treatment 530 on the value of the condition 8 of an internal structure of hair 5.

Based on the information that is available from the application of the method 200 of the invention, it is possible to adjust the hair treatment 530 recommendations made to a user and determine which products or hair treatments 530 have the strongest and most desired effect on the condition 8 of the internal structure of their hair 5.

## Claims

1. A method (200) for determining a condition (8) of the internal structure of hair and the degree of hair damage, the method (200) comprising:
- while exposing a hair sample (30) of the hair (5) to at least one among light (1), stress (4), elongation (3) or heat (2), recording (201) a signal (61) from at least one part of the light that has interacted with the hair sample (30) or a signal (6) from the response of the hair sample to that stress, elongation or heat;
- comparing (202) the recorded signal (6; 61) with a first calibration model (7) and determining the condition (8) of the internal structure of hair based on the comparison; Wherein the calibration model (8) is obtained by:
- determining the condition of the internal structure of a plurality of calibration hair samples using a method that measures internal constituents of hair fibers;
- exposing each of said plurality of calibration hair samples to at least one among light, stress, elongation or heat, recording a signal from at least one part of the light that has interacted with each calibration hair sample or a signal from the response of the hair sample to that stress, elongation or heat;
- establishing the first calibration model by determining a correlation between the recorded signals and the determined condition of the internal structure of the plurality of calibration hair samples;
**characterized in that** the method further comprises:
obtaining a second calibration (71) model that establishes a correlation between a determined degree of hair damage of the calibration hair samples and signals recorded from at least one part of the light that has interacted with calibration hair samples or signals corresponding to a response of hair to stress, elongation or heat while exposing calibration hair samples of the hair to that light, stress, elongation or heat;
wherein the signal recorded while exposing the hair sample (30) of the hair (5) to at least one among light, stress, elongation or heat is compared to the second calibration model (71) to determine a degree of hair damage (81) of the hair sample.

2. The method (200) of claim 1, wherein the condition of the internal structure of hair is at least one among: the degree of hair damage, the presence of external chemical compounds that are on or in hair, the presence and type of chemical compounds that are on or in hair, a moisture content of hair.

3. The method (200) of claim 1 or 2, wherein the independent method that measures internal constituents of hair fibers is at least one among: differential scanning calorimetry applied to hair, a mass spectrometry of hair, electron microscopy of hair, stress-strain measurements, fatigue measurements, elongation measurements, hair hydrophobicity or swelling detection, measurements of non-linear optical effects arising in hair such as birefringence of waves, attenuated total reflectance, Fourier Transform Interferometry.

4. The method (200) according to any one of the preceding claims, wherein the recorded signal is at least one among: an infrared light spectrum, a near-infrared light spectrum, a combination of wavelengths forming a hyperspectral signal, a Fourier Transform Interferometry signal, an image of hair, a differential scanning calorimetry applied to hair, stress-strain measurements on hair, elongation measurements conducted on hair, a birefringence signal scattered by hair, a signal related to a vibration of hair, a signal related to the response of hair to ultrasounds, a pH value measured on hair, an electrical conductivity of hair, a signal related to surface charge of hair.

5. The method (200) according to claim 1, wherein the degree of hair damage is determined using at least one among High Performance Liquid Chromatography and a chromatographic method that determines a degree of cysteic acid content of the calibration hair samples.

6. The method (200) according to claim 1, further comprising:
- determining a first value (512) of the condition of the internal structure of hair and a first value of the degree of hair damage prior to applying a hair treatment to the hair;
- determining a second value of the condition of the internal structure of hair and a second value of the degree of hair damage after application of the hair treatment;
- comparing the difference between the second value of the degree of hair damage and the first value of the degree of hair damage to the difference between the second value of the condition of the internal structure of hair and the first value of the condition of the internal structure of hair;
- determining, based on said comparison, a specific contribution of the application of the hair treatment to the condition of the hair.

7. The method (200) according to claim 1, further comprising:
- determining a relationship between values of the condition of the internal structure of hair obtained using the first calibration model and values of the degree of hair damage obtained using the second calibration model;
- determining a value of the condition of the internal structure of hair and a value of the degree of hair damage after application of the hair treatment;
- using the determined relationship to transform the value of the degree of hair damage into a corresponding equivalent value of the condition of the internal structure of hair;
- subtracting from the determined value of the condition of the internal structure of hair the corresponding equivalent value to estimate the additional contribution of the application of the hair treatment on the value of the condition of the internal structure of hair.

8. The method (200) according to claim 1 further comprising:
- determining a first value (512) of the condition of the internal structure of hair prior to applying a hair treatment to the hair;
- determining a second value (542) of the condition of the internal structure of hair after application of the hair treatment;
- comparing the difference between the second value of the condition of the internal structure of hair and the first value of the condition of the internal structure of hair;
- determining, based on said comparison, a specific contribution of the application of the hair treatment to the condition of the hair.

9. The method (200) according to any one of the preceding claims, further comprising:
- providing a recommendation of a hair treatment based on the determined condition of the internal structure of hair, the recommended hair treatment being selected from a database associating hair treatments to a quantified effect of the hair treatment on improving a condition of hair after application of said hair treatment based on an initial condition of the internal structure of hair.

10. A device (100) for determining a condition of the internal structure of hair (5), the device (100) comprising:
- a detector (102) configured to record a signal (61) from at least one part of light that has interacted with a hair sample (30) of the hair (5) or to record a signal (6) from the response of the hair sample (30) to at least one among stress, elongation or heat while the hair sample (30) is exposed to at least one among light, stress, elongation or heat;
- a processor (104, 111) configured to compare the recorded signal (6,61) with a first calibration model (7) and to determine the condition (8) of the internal structure of hair (5) based on the comparison; the processor being further configured to obtain the first calibration model, wherein the first calibration model comprises a correlation between:
- signals recorded by exposing each of a plurality of calibration hair samples to at least one among light, stress, elongation or heat and recording a signal from at least one part of the light that has interacted with each calibration hair sample or a signal from the response of the hair sample to that stress, elongation or heat, and
- determined conditions of the internal structure of the plurality of calibration hair samples using a method that measures internal constituents of hair fibers;
- a processor (104, 111) further configured to compare the recorded signal with a second calibration model (71) and to determine a degree of hair damage (81) of the hair sample based on the comparison; the processor being further configured to obtain the second calibration model, wherein the second calibration model comprises a correlation between:
a determined degree of hair damage of the calibration hair samples and signals recorded from at least one part of the light that has interacted with calibration hair samples or signals corresponding to a response of hair to stress, elongation or heat while exposing calibration hair samples of the hair to that light, stress, elongation or heat.

11. The device (100) of claim 10, wherein the detector (102) comprises at least one among: an infrared sensor, a near infrared sensor, a sensor capable of acquiring or reconstructing a hyperspectral signal, a heat capacitance sensor configured to acquire a differential scanning calorimetry signal, a camera configured to acquire an image of the hair.

12. The device (100) according to any one of claims 10 or 11, wherein the device is at least one among: a handheld device configured to grasp a strand of hair between two portions of the handheld device, a mobile terminal.

13. Computer program product comprising program instructions configured to implement a method for determining a condition of the internal structure of hair, the method comprising:
- obtaining a recorded signal corresponding to at least one part of the light that has interacted with a hair sample of hair or a recorded signal corresponding to the response of hair to stress, elongation or heat while exposing the hair sample to at least one among light, stress, elongation or heat;
- comparing the recorded signal with a first calibration model and determining the condition of the internal structure of hair based on the comparison;
the method further comprising obtaining the first calibration model comprising a correlation between:
- signals recorded by exposing each of a plurality of calibration hair samples to at least one among light, stress, elongation or heat and recording a signal from at least one part of the light that has interacted with each calibration hair sample or a signal from the response of the hair sample to that stress, elongation or heat, and
- determined conditions of the internal structure of the plurality of calibration hair samples using a method that measures internal constituents of hair fibers;
- comparing the recorded signal to a second calibration model (71) and determining a degree of hair damage (81) of the hair sample based on the comparison;
the method further comprising obtaining the second calibratior model comprising a correlation between:
a determined degree of hair damage of the calibration hair samples and signals recorded from at least one part of the light that has interacted with calibration hair samples or signals corresponding to a response of hair to stress, elongation or heat while exposing calibration hair samples of the hair to that light, stress, elongation or heat.

## Patentansprüche

1. Verfahren (200) zur Bestimmung eines Zustands (8) der inneren Struktur von Haaren und des Ausmaßes der Haarschädigung, wobei das Verfahren (200) umfasst:
- während einer Haarprobe (30) des Haares (5) mindestens einer der folgenden Einwirkungen ausgesetzt wird: Licht (1), Belastung (4), Dehnung (3) oder Wärme (2) aussetzt, das Aufzeichnen (201) eines Signals (61) von mindestens einem Teil des Lichts, das mit der Haarprobe (30) in Wechselwirkung getreten ist, oder eines Signals (6) aus der Reaktion der Haarprobe auf diese Belastung, Dehnung oder Wärme;
- Vergleichen (202) des aufgezeichneten Signals (6; 61) mit einem ersten Kalibrierungsmodell (7) und Bestimmen des Zustands (8) der inneren Struktur des Haares auf der Grundlage des Vergleichs;
wobei das Kalibrierungsmodell (8) erhalten wird durch:
- Bestimmung des Zustands der inneren Struktur einer Vielzahl von Kalibrierungshaarproben unter Verwendung eines Verfahrens, das die inneren Bestandteile von Haarfasern misst;
- Aussetzen jeder der mehreren Kalibrierungshaarproben mindestens einem der folgenden Einflüsse: Licht, Belastung, Dehnung oder Wärme, Aufzeichnen eines Signals von mindestens einem Teil des Lichts, das mit jeder Kalibrierungshaarprobe in Wechselwirkung getreten ist, oder eines Signals aus der Reaktion der Haarprobe auf diese Belastung, Dehnung oder Wärme;
- Erstellen des ersten Kalibrierungsmodells durch Bestimmen einer Korrelation zwischen den aufgezeichneten Signalen und dem ermittelten Zustand der inneren Struktur der mehreren Kalibrierungshaarproben;
Erhalten eines zweiten Kalibrierungsmodells (71), das eine Korrelation zwischen einem ermittelten Grad der Haarschädigung der Kalibrierungshaarproben und Signalen herstellt, die von mindestens einem Teil des Lichts aufgezeichnet wurden, das mit den Kalibrierungshaarproben in Wechselwirkung getreten ist, oder von Signalen, die einer Reaktion des Haares auf Belastung, Dehnung oder Wärme entsprechen, während die Kalibrierungshaarproben des Haares diesem Licht, dieser Belastung, dieser Dehnung oder dieser Wärme ausgesetzt werden;
wobei das Signal, das während der Einwirkung von Licht, Belastung, Dehnung oder Wärme auf die Haarprobe (30) des Haares (5) aufgezeichnet wurde, mit dem zweiten Kalibrierungsmodell (71) verglichen wird, um einen Grad der Haarschädigung (81) der Haarprobe zu bestimmen.

2. Verfahren (200) nach Anspruch 1, wobei der Zustand der inneren Struktur des Haares mindestens eines der folgenden ist: der Grad der Haarschädigung, das Vorhandensein von externen chemischen Verbindungen, die sich auf oder im Haar befinden, das Vorhandensein und die Art von chemischen Verbindungen, die sich auf oder im Haar befinden, ein Feuchtigkeitsgehalt des Haares.

3. Verfahren (200) nach Anspruch 1 oder 2, wobei das unabhängige Verfahren, das die Volumeneigenschaften des Haares misst, mindestens eines der folgenden ist: Differential-Scanning-Kalorimetrie, die auf das Haar angewendet wird, eine Massenspektrometrie des Haares, Elektronenmikroskopie des Haares, Spannungs-Dehnungs-Messungen, Ermüdungsmessungen, Dehnungsmessungen, die Bestimmung der Hydrophobie oder des Quellverhaltens des Haares, Messungen nichtlinearer optischer Effekte, die im Haar auftreten, wie beispielsweise Doppelbrechung von Wellen, abgeschwächte Totalreflexion, Fourier-Transform-Interferometrie.

4. Verfahren (200) gemäß einem der vorstehenden Ansprüche, wobei das aufgezeichnete Signal mindestens eines der folgenden ist:
ein Infrarotlichtspektrum, ein Nahinfrarotlichtspektrum, eine Kombination von Wellenlängen, die ein hyperspektrales Signal bilden, ein Fourier-Transform-Interferometriesignal, ein Bild des Haares, eine auf das Haar angewandte Differential-Scanning-Kalorimetrie, Spannungs-Dehnungs-Messungen am Haar, Dehnungsmessungen am Haar, einem durch Haar gestreuten Doppelbrechungssignal, einem Signal, das sich auf eine Schwingung von Haar bezieht, einem Signal, das sich auf die Reaktion von Haar auf Ultraschall bezieht, einem an Haar gemessenen pH-Wert, einer elektrischen Leitfähigkeit von Haar, einem Signal, das sich auf die Oberflächenladung von Haar bezieht.

5. Verfahren (200) nach Anspruch 1, wobei der Grad der Haarschädigung unter Verwendung mindestens eines der folgenden Verfahren bestimmt wird: Hochleistungsflüssigkeitschromatographie und ein chromatographisches Verfahren zur Bestimmung des Cysteinsäuregehalts der Kalibrierhaarproben.

6. Verfahren (200) nach Anspruch 1, das ferner umfasst:
- Bestimmen eines ersten Wertes (512) des Zustands der inneren Struktur des Haares und eines ersten Wertes des Ausmaßes der Haarschädigung vor dem Auftragen einer Haarbehandlung auf das Haar;
- Bestimmung eines zweiten Wertes für den Zustand der inneren Struktur des Haares und eines zweiten Wertes für den Grad der Haarschädigung nach der Anwendung der Haarbehandlung;
- Vergleichen der Differenz zwischen dem zweiten Wert des Ausmaßes der Haarschädigung und dem ersten Wert des Ausmaßes der Haarschädigung mit der Differenz zwischen dem zweiten Wert des Zustands der inneren Haarstruktur und dem ersten Wert des Zustands der inneren Haarstruktur;
- Bestimmen eines spezifischen Beitrags der Anwendung der Haarbehandlung zum Zustand des Haares auf der Grundlage des genannten Vergleichs.

7. Verfahren (200) nach Anspruch 1, das ferner umfasst:
- Bestimmen einer Beziehung zwischen Werten des Zustands der inneren Haarstruktur, die unter Verwendung des ersten Kalibrierungsmodells erhalten wurden, und Werten des Grades der Haarschädigung, die unter Verwendung des zweiten Kalibrierungsmodells erhalten wurden;
- Bestimmen eines Wertes für den Zustand der inneren Haarstruktur und eines Wertes für den Grad der Haarschädigung nach Anwendung der Haarbehandlung;
- Verwenden der ermittelten Beziehung, um den Wert des Ausmaßes der Haarschädigung in einen entsprechenden äquivalenten Wert des Zustands der inneren Haarstruktur umzuwandeln;
- Subtrahieren des entsprechenden Äquivalenzwerts von dem ermittelten Wert des Zustands der inneren Haarstruktur, um den zusätzlichen Beitrag der Anwendung der Haarbehandlung auf den Wert des Zustands der inneren Haarstruktur abzuschätzen.

8. Das Verfahren (200) gemäß Anspruch 1, das ferner umfasst:
- Bestimmen eines ersten Wertes (512) des Zustands der inneren Haarstruktur vor der Anwendung einer Haarbehandlung auf das Haar;
- Bestimmen eines zweiten Wertes (542) für den Zustand der inneren Haarstruktur nach der Anwendung der Haarbehandlung;
- Vergleichen der Differenz zwischen dem zweiten Wert des Zustands der inneren Haarstruktur und dem ersten Wert des Zustands der inneren Haarstruktur;
- Bestimmen, basierend auf dem Vergleich, eines spezifischen Beitrags der Anwendung der Haarbehandlung zum Zustand des Haares.

9. Verfahren (200) gemäß einem der vorstehenden Ansprüche, das ferner umfasst:
- Bereitstellen einer Empfehlung für eine Haarbehandlung auf der Grundlage des ermittelten Zustands der inneren Struktur des Haares, wobei die empfohlene Haarbehandlung aus einer Datenbank ausgewählt wird, die Haarbehandlungen mit einer quantifizierten Wirkung der Haarbehandlung auf die Verbesserung eines Zustands des Haares nach Anwendung der Haarbehandlung auf der Grundlage eines Ausgangszustands der inneren Struktur des Haares verknüpft.

10. Vorrichtung (100) zur Bestimmung eines Zustands der inneren Struktur von Haaren (5), wobei die Vorrichtung (100) umfasst:
- einen Detektor (102), der so ausgelegt ist, dass er ein Signal (61) von mindestens einem Teil des Lichts aufzeichnet, das mit einer Haarprobe (30) des Haares (5) in Wechselwirkung getreten ist, oder ein Signal (6) aus der Reaktion der Haarprobe (30) auf mindestens eine der folgenden Einwirkungen aufzeichnet: Belastung, Dehnung oder Wärme, während die Haarprobe (30) mindestens einer der folgenden Einwirkungen ausgesetzt ist: Licht, Belastung, Dehnung oder Wärme ausgesetzt ist;
- einen Prozessor (104, 111), der so konfiguriert ist, dass er das aufgezeichnete Signal (6, 61) mit einem ersten Kalibrierungsmodell (7) vergleicht und auf der Grundlage des Vergleichs den Zustand (8) der inneren Struktur des Haares (5) bestimmt; wobei der Prozessor weiter t, so konfiguriert zu sein, dass er das erste Kalibrierungsmodell erhält, wobei das erste Kalibrierungsmodell eine Korrelation zwischen folgenden Größen umfasst:
- Signalen, die aufgezeichnet wurden, indem jede einer Vielzahl von Kalibrierungshaarproben mindestens einem der Faktoren Licht, Belastung, Dehnung oder Wärme ausgesetzt wurde und ein Signal von mindestens einem Teil des Lichts, das mit jeder Kalibrierungshaarprobe in Wechselwirkung getreten ist, oder ein Signal aus der Reaktion der Haarprobe auf diese Belastung, Dehnung oder Wärme aufgezeichnet wurde, und
- ermittelten Zuständen der inneren Struktur der Vielzahl von Kalibrierungshaarproben unter Verwendung eines Verfahrens, das innere Bestandteile von Haarfasern misst;
- einem Prozessor (104, 111), der ferner so konfiguriert ist, dass er das aufgezeichnete Signal mit dem zweiten Kalibrierungsmodell (71) vergleicht, um einen Grad der Haarschädigung (81) der Haarprobe zu bestimmen, wobei das zweite Kalibrierungsmodell eine Korrelation umfasst zwischen:
einem ermittelten Grad der Haarschädigung der Kalibrierungshaarproben und Signalen, die von mindestens einem Teil des Lichts aufgezeichnet wurden, das mit den Kalibrierungshaarproben in Wechselwirkung getreten ist, oder Signalen, die einer Reaktion des Haares auf Belastung, Dehnung oder Wärme entsprechen, während die Kalibrierungshaarproben dem Licht, der Belastung, der Dehnung oder der Wärme ausgesetzt wurden.

11. Vorrichtung (100) nach Anspruch 10, wobei der Detektor (102) mindestens eines der folgenden Elemente umfasst: einen Infrarotsensor, einen Nahinfrarotsensor, einen Sensor, der in der Lage ist, ein hyperspektrales Signal zu erfassen oder zu rekonstruieren, einen Wärmekapazitätssensor, der so konfiguriert ist, dass er ein Differential-Scanning-Kalorimetrie-Signal erfasst, eine Kamera, die so konfiguriert ist, dass sie ein Bild des Haares erfasst.

12. Vorrichtung (100) nach einem der Ansprüche 10 oder 11, wobei die Vorrichtung mindestens eines der folgenden Elemente umfasst: ein Handgerät, das so konfiguriert ist, dass es eine Haarsträhne zwischen zwei Teilen des Handgeräts erfasst, ein mobiles Endgerät.

13. Computerprogrammprodukt, das Programmanweisungen umfasst, die so konfiguriert sind, dass sie ein Verfahren zur en Bestimmung eines Zustands der inneren Struktur von Haaren implementieren, wobei das Verfahren umfasst:
- Erfassen eines aufgezeichneten Signals, das mindestens einem Teil des Lichts entspricht, das mit einer Haarprobe in Wechselwirkung getreten ist, oder eines aufgezeichneten Signals, das der Reaktion des Haares auf Belastung, Dehnung oder Wärme entspricht, während die Haarprobe mindestens einem der Faktoren Licht, Belastung, Dehnung oder Wärme ausgesetzt wird;
- das Erfassen des aufgezeichneten Signals mit einem ersten Kalibrierungsmodell und das Bestimmen des Zustands der inneren Struktur des Haares auf der Grundlage des Vergleichs; wobei das Verfahren ferner das Erhalten eines ersten Kalibrierungsmodells umfasst, das eine Korrelation zwischen folgenden Größen umfasst:
- Signale, die aufgezeichnet wurden, indem jede einer Vielzahl von Kalibrierungshaarproben mindestens einer der folgenden Einwirkungen ausgesetzt wurde: Licht, Belastung, Dehnung oder Wärme, und indem ein Signal von mindestens einem Teil des Lichts, das mit jeder Kalibrierungshaarprobe in Wechselwirkung getreten ist, oder ein Signal aus der Reaktion der Haarprobe auf diese Belastung, Dehnung oder Wärme aufgezeichnet wurde, und
- Bestimmung der Zustände der inneren Struktur der mehreren Kalibrierungshaarproben unter Verwendung eines Verfahrens, das die inneren Bestandteile von Haarfasern misst;
- Vergleichen des aufgezeichneten Signals mit dem zweiten Kalibrierungsmodell (71), um auf der Grundlage des Vergleichs einen Grad der Haarschädigung (81) der Haarprobe zu bestimmen;
wobei das Verfahren ferner das Ermitteln eines zweiten Kalibrierungsmodells umfasst, das eine Korrelation zwischen folgenden Größen beinhaltet:
einem ermittelten Grad der Haarschädigung der Kalibrierungshaarproben und Signalen, die von mindestens einem Teil des Lichts aufgezeichnet wurden, das mit den Kalibrierungshaarproben in Wechselwirkung getreten ist, oder Signalen, die einer Reaktion des Haares auf Belastung, Dehnung oder Wärme entsprechen, während die Kalibrierungshaarproben dem Licht, der Belastung, der Dehnung oder der Wärme ausgesetzt wurden.

## Revendications

1. Procédé (200) permettant de déterminer l'état (8) de la structure interne d'un cheveu et le degré d'endommagement de celui-ci, le procédé (200) comprenant :
- tout en exposant un échantillon de cheveux (30) des cheveux (5) à au moins l'un parmi la lumière (1), la contrainte (4), d'étirement (3) ou de chaleur (2), l'enregistrement (201) d'un signal (61) provenant d'au moins une partie de la lumière ayant interagi avec l'échantillon de cheveux (30) ou d'un signal (6) provenant de la réponse de l'échantillon de cheveux à cette contrainte, cet étirement ou cette chaleur ;
- comparer (202) le signal enregistré (6 ; 61) à un premier modèle d'étalonnage (7) et déterminer l'état (8) de la structure interne du cheveu sur la base de cette comparaison ;
dans lequel le modèle d'étalonnage (8) est obtenu par :
- la détermination de l'état de la structure interne d'une pluralité d'échantillons de cheveux d'étalonnage à l'aide d'une méthode qui mesure les constituants internes des fibres capillaires ;
- l'exposition de chacun desdits échantillons de cheveux d'étalonnage à au moins l'un parmi la lumière, une contrainte, un allongement ou la chaleur, l'enregistrement d'un signal provenant d'au moins une partie de la lumière ayant interagi avec chaque échantillon de cheveux d'étalonnage ou d'un signal provenant de la réponse de l'échantillon de cheveux à cette contrainte, cet allongement ou cette chaleur ;
- établir le premier modèle d'étalonnage en déterminant une corrélation entre les signaux enregistrés et l'état déterminé de la structure interne de la pluralité d'échantillons de cheveux d'étalonnage ;
obtenir un deuxième modèle d'étalonnage (71) qui établit une corrélation entre un degré déterminé de détérioration des cheveux des échantillons de cheveux d'étalonnage et des signaux enregistrés à partir d'au moins une partie de la lumière ayant interagi avec les échantillons de cheveux d'étalonnage ou des signaux correspondant à une réponse des cheveux à la contrainte, à l'xml-ph-0000@deepl.internalu à la chaleur tout en exposant les échantillons de cheveux d'étalonnage des cheveux à ladite lumière, contrainte, xml-ph-0000@deepl.internalu chaleur ;
dans lequel le signal enregistré lors de l'exposition de l'échantillon de cheveux (30) des cheveux (5) à au moins l'un parmi la lumière, le stress, l' , l'allongement ou la chaleur est comparé au deuxième modèle d'étalonnage (71) afin de déterminer un degré de détérioration des cheveux (81) de l'échantillon de cheveux.

2. Procédé (200) selon la revendication 1, dans lequel l'état de la structure interne du cheveu est au moins l'un parmi : le degré de détérioration du cheveu, la présence de composés chimiques externes qui se trouvent sur ou dans le cheveu, la présence et le type de composés chimiques qui se trouvent sur ou dans le cheveu, une teneur en humidité du cheveu.

3. Procédé (200) selon la revendication 1 ou 2, dans lequel le procédé indépendant qui mesure les propriétés globales du cheveu est au moins l'un parmi : la calorimétrie à balayage différentiel appliquée aux cheveux, la spectrométrie de masse des cheveux, la microscopie électronique des cheveux, les mesures de contrainte-déformation, les mesures de fatigue, les mesures d'allongement, la détection de l'hydrophobicité ou du gonflement des cheveux, les mesures d'effets optiques non linéaires survenant dans les cheveux, tels que la biréfringence des ondes, la réflexion totale atténuée, l'interférométrie à transformée de Fourier.

4. Procédé (200) selon l'une quelconque des revendications précédentes, dans lequel le signal enregistré est au moins l'un des éléments suivants : un spectre de lumière infrarouge, un spectre de lumière proche infrarouge, une combinaison de longueurs d'onde formant un signal hyperspectral, un signal d'interférométrie par transformée de Fourier, une image de cheveux, une calorimétrie à balayage différentiel appliquée aux cheveux, des mesures de contrainte-déformation sur les cheveux, des mesures d'allongement effectuées sur les cheveux, un signal de biréfringence diffusé par les cheveux, un signal lié à une vibration des cheveux, un signal lié à la réponse des cheveux aux ultrasons, une valeur de pH mesurée sur les cheveux, une conductivité électrique des cheveux, un signal lié à la charge superficielle des cheveux.

5. Procédé (200) selon la revendication 1, dans lequel le degré de détérioration des cheveux est déterminé à l'aide d'au moins l'une parmi la chromatographie liquide haute performance et une méthode d' e chromatographique qui détermine le degré de teneur en acide cystéique des échantillons de cheveux d'étalonnage.

6. Procédé (200) selon la revendication 1, comprenant en outre :
- la détermination d'une première valeur (512) de l'état de la structure interne du cheveu et d'une première valeur du degré de détérioration du cheveu avant l'application d'un traitement capillaire sur le cheveu ;
- la détermination d'une deuxième valeur de l'état de la structure interne des cheveux et d'une deuxième valeur du degré de détérioration des cheveux après l'application du traitement capillaire ;
- la comparaison de la différence entre la deuxième valeur du degré de détérioration des cheveux et la première valeur du degré de détérioration des cheveux avec la différence entre la deuxième valeur de l'état de la structure interne des cheveux et la première valeur de l'état de la structure interne des cheveux ;
- déterminer, sur la base de ladite comparaison, une contribution spécifique de l'application du traitement capillaire à l'état des cheveux.

7. Procédé (200) selon la revendication 1, comprenant en outre :
- la détermination d'une relation entre les valeurs de l'état de la structure interne des cheveux obtenues à l'aide du premier modèle d'étalonnage et les valeurs du degré de détérioration des cheveux obtenues à l'aide du deuxième modèle d'étalonnage ;
- déterminer une valeur de l'état de la structure interne des cheveux et une valeur du degré de détérioration des cheveux après application du traitement capillaire ;
- l'utilisation de la relation déterminée pour transformer la valeur du degré de détérioration des cheveux en une valeur équivalente correspondante de l'état de la structure interne des cheveux ;
- soustraire de la valeur déterminée de l'état de la structure interne du cheveu la valeur équivalente correspondante afin d'estimer la contribution supplémentaire de l'application du traitement capillaire sur la valeur de l'état de la structure interne du cheveu.

8. Le procédé (200) selon la revendication 1, comprenant en outre :
- la détermination d'une première valeur (512) de l'état de la structure interne des cheveux avant l'application d'un traitement capillaire sur les cheveux ;
- déterminer une deuxième valeur (542) de l'état de la structure interne du cheveu après l'application du traitement capillaire ;
- la comparaison de la différence entre la deuxième valeur de l'état de la structure interne des cheveux et la première valeur de l'état de la structure interne des cheveux ;
- déterminer, sur la base de ladite comparaison, une contribution spécifique de l'application du traitement capillaire à l'état des cheveux.

9. Procédé (200) selon l'une quelconque des revendications précédentes, comprenant en outre :
- fournir une recommandation de traitement capillaire sur la base de l'état déterminé de la structure interne du cheveu, le traitement capillaire recommandé étant sélectionné à partir d'une base de données associant des traitements capillaires à un effet quantifié dudit traitement capillaire sur l'amélioration de l'état du cheveu après application dudit traitement capillaire, sur la base d'un état initial de la structure interne du cheveu.

10. Dispositif (100) destiné à déterminer l'état de la structure interne des cheveux (5), le dispositif (100) comprenant :
- un détecteur (102) conçu pour enregistrer un signal (61) provenant d'au moins une partie de la lumière ayant interagi avec un échantillon de cheveu (30) du cheveu (5) ou pour enregistrer un signal (6) résultant de la réaction de l'échantillon de cheveu (30) à au moins l'un des facteurs suivants : contrainte, allongement ou chaleur, tandis que l'échantillon de cheveu (30) est exposé à au moins l'un des facteurs suivants : lumière, contrainte, d'allongement ou de chaleur ;
- un processeur (104, 111) configuré pour comparer le signal enregistré (6, 61) à un premier modèle d'étalonnage (7) et pour déterminer l'état (8) de la structure interne du cheveu (5) sur la base de cette comparaison ; le processeur étant en outre configuré pour obtenir le premier modèle d'étalonnage, dans lequel le premier modèle d'étalonnage comprend une corrélation entre :
- des signaux enregistrés en exposant chacun d'une pluralité d'échantillons de cheveux d'étalonnage à au moins l'un parmi la lumière, la contrainte, l'allongement ou la chaleur et en enregistrant un signal provenant d'au moins une partie de la lumière ayant interagi avec chaque échantillon de cheveux d'étalonnage ou un signal provenant de la réponse de l'échantillon de cheveux à cette contrainte, cet allongement ou cette chaleur, et
- des conditions déterminées de la structure interne de la pluralité d'échantillons de cheveux d'étalonnage à l'aide d'un procédé qui mesure les constituants internes des fibres capillaires ;
- un processeur (104, 111) configuré en outre pour comparer le signal enregistré au deuxième modèle d'étalonnage (71) afin de déterminer un degré de détérioration capillaire (81) de l'échantillon de cheveux, dans lequel le deuxième modèle d'étalonnage comprend une corrélation entre :
un degré déterminé de détérioration des cheveux des échantillons de cheveux d'étalonnage et des signaux enregistrés à partir d'au moins une partie de la lumière ayant interagi avec les échantillons de cheveux d'étalonnage ou des signaux correspondant à une réponse des cheveux à une contrainte, à un étirement ou à la chaleur lors de l'exposition des échantillons de cheveux d'étalonnage à ladite lumière, contrainte, étirement ou chaleur.

11. Le dispositif (100) selon la revendication 10, dans lequel le détecteur (102) comprend au moins l'un parmi : un capteur infrarouge, un capteur proche infrarouge, un capteur capable d'acquérir ou de reconstruire un signal hyperspectral, un capteur de capacité thermique configuré pour acquérir un signal de calorimétrie à balayage différentiel, une caméra configurée pour acquérir une image des cheveux.

12. Dispositif (100) selon l'une quelconque des revendications 10 ou 11, dans lequel le dispositif est au moins l'un parmi : un dispositif portatif configuré pour saisir une mèche de cheveux entre deux parties du dispositif portatif, un terminal mobile.

13. Produit logiciel comprenant des instructions de programme configurées pour mettre en œuvre un procédé d' , déterminant un état de la structure interne d'un cheveu, le procédé comprenant :
- l'obtention d'un signal enregistré correspondant à au moins une partie de la lumière ayant interagi avec un échantillon de cheveux ou d'un signal enregistré correspondant à la réponse des cheveux à une contrainte, à un étirement ou à la chaleur, tout en exposant l'échantillon de cheveux à au moins l'un parmi la lumière, la contrainte, l'étirement ou la chaleur ;
- la comparaison du signal enregistré avec un premier modèle d'étalonnage et la détermination de l'état de la structure interne du cheveu sur la base de cette comparaison ;
la méthode comprenant en outre l'obtention d'un premier modèle d'étalonnage comprenant une corrélation entre :
- des signaux enregistrés en soumettant chacun d'une pluralité d'échantillons de cheveux d'étalonnage à au moins l'un des facteurs suivants : lumière, contrainte, allongement ou chaleur, et en enregistrant un signal provenant d'au moins une partie de la lumière ayant interagi avec chaque échantillon de cheveux d'étalonnage, ou un signal résultant de la réponse de l'échantillon de cheveux à cette contrainte, cet allongement ou cette chaleur, et
- déterminer les conditions de la structure interne de la pluralité d'échantillons de cheveux d'étalonnage à l'aide d'une méthode qui mesure les constituants internes des fibres capillaires ;
- comparer le signal enregistré au deuxième modèle d'étalonnage (71) afin de déterminer un degré de détérioration (81) de l'échantillon de cheveu sur la base de la comparaison ;
la méthode comprenant en outre l'obtention d'un deuxième modèle d'étalonnage comprenant une corrélation entre :
un degré déterminé de détérioration des cheveux des échantillons de cheveux d'étalonnage et des signaux enregistrés à partir d'au moins une partie de la lumière ayant interagi avec les échantillons de cheveux d'étalonnage ou des signaux correspondant à une réponse des cheveux à une contrainte, à un allongement ou à la chaleur tout en exposant les échantillons de cheveux d'étalonnage à cette lumière, cette contrainte, cet allongement ou cette chaleur.
